# EUROPEAN PATENT APPLICATION

(11) **EP 3 382 986 A1**
(43) Date of publication of application: **03.10.2018**
(21) Application number: 18161466.0
(22) Date of filing: 13.03.2018
(51) Int. Cl.: H04L 29/06, H04N 7/15, G06F 3/0488

(54) **APPROACH FOR DISPLAYING INFORMATION ON INTERACTIVE WHITEBOARD (IWB) APPLIANCES**

(30) Priority: 31.03.2017 US 201715476186
(71) Applicant: Ricoh Company, Ltd., Tokyo 143-8555 (JP)
(72) Inventor: KNODT, Kurt, Campbell, CA 95008 (US)
(74) Representative: Schwabe - Sandmair - Marx

(57) **Abstract**

An IWB appliance includes a tracking board application that causes first data to be displayed on a display of the IWB appliance in a display-only manner, and second data to be displayed on the display of the IWB appliance in an editable manner. The first data and the second data are displayed on one or more other IWB appliances that are different than the IWB appliance, and at least a portion of the second data was edited at an IWB appliance from the one or more other IWB appliances. Embodiments include a timeline view that allows a user to view prior versions of the first and second data that correspond to particular dates and/or times, support for user authentication, views, copy and paste of edited information, and mobile devices.

## Description

### BACKGROUND OF THE INVENTION

### 1. Field of the Invention

Embodiments relate generally to displaying information on interactive whiteboard (IWB) appliances.

### 2. Description of the Related Art

The approaches described in this section are approaches that could be pursued, but not necessarily approaches that have been previously conceived or pursued. Therefore, unless otherwise indicated, it should not be assumed that any of the approaches described in this section qualify as prior art merely by virtue of their inclusion in this section.

Conventional dry-erase whiteboards have been in widespread use in business organizations for many years. They are often used during business meetings to share and collaborate on information. They are also commonly used to track changes in information over time. For example, manufacturing businesses use dry-erase whiteboards to track the status of products being manufactured. As another example, healthcare facilities use dry-erase whiteboards to track patient information, such as room assignments and assigned clinicians. Dry-erase whiteboards are easy to install and use, are inexpensive, and are available in a wide variety of sizes.

Despite these many benefits, dry-erase whiteboards have several limitations. First, individuals must walk to, and be physically present at, dry-erase whiteboards to view and change the information, and people in remote locations do not have access to the information. Also, there is no change control or history. Anyone can walk up and change information on a dry-erase whiteboard, with or without authorization, and the information that was changed is forever lost and cannot be retrieved. In addition, since information on dry-erase whiteboards must be written by hand, the information may not always be legible, and the information cannot be obtained from, or synchronized with, an electronic information system, such as a database system, or in the medical context, an Electronic Health Record (EHR) system.

### SUMMARY OF THE INVENTION

An interactive whiteboard appliance (IWB) comprises a display, one or more processors, and a tracking board application. The tracking board application is configured to cause first data to be displayed on the display of the IWB appliance in a display-only manner that allows a user of the IWB appliance to view, but not edit, the first data. The tracking board application is also configured to cause second data to be displayed on the display of the IWB appliance, concurrently with the first data, in an editable manner that allows the user of the IWB appliance to view and edit the second data. The first data and the second data are displayed on one or more other IWB appliances that are different than the IWB appliance, and at least a portion of the second data was received by the IWB appliance from the one or more other IWB appliances.

### BRIEF DESCRIPTION OF THE DRAWINGS

In the figures of the accompanying drawings like reference numerals refer to similar elements.
FIG. 1 is a block diagram that depicts an arrangement for displaying information on IWB appliances.
FIG. 2A depicts an example healthcare tracking board generated by healthcare tracking board applications that displays healthcare information.
FIG. 2B depicts a "Room Map" view in response to a user selecting the "Room Map" control from controls.
FIG. 2C depicts a healthcare tracking board in response to a user selecting the "Timeline View" option from view controls.
FIG. 3 depicts a message ladder diagram that shows example message flows between the various elements of FIG. 1.
FIG. 4 is a block diagram that depicts an example computer system upon which embodiments may be implemented.

### DETAILED DESCRIPTION OF THE PREFERRED EMBODIMENTS

In the following description, for the purposes of explanation, numerous specific details are set forth in order to provide a thorough understanding of the embodiments. It will be apparent, however, to one skilled in the art that the embodiments may be practiced without these specific details. In other instances, well-known structures and devices are shown in block diagram form in order to avoid unnecessarily obscuring the embodiments.
**I. Overview**
**II. System Architecture**
   **Overview**
   **IWB Appliances**
   **IWB Server**
   **EHR System**
   **Client Devices**
**III. Displaying information on IWB Appliances**
**IV. Timeline Views**
**V. Updating Healthcare Data on EHR System**
**VI. Implementation Examples**

### I. Overview

An approach is provided for displaying information on interactive whiteboard (IWB) appliances. According to the approach, an IWB appliance includes a tracking board application that causes first data to be displayed on a display of the IWB appliance in a display-only manner that allows a user of the IWB appliance to view, but not edit, the first data. The tracking board application also causes second data to be displayed on the display of the IWB appliance, concurrent with the display of the first data on the display of the IWB appliance, in an editable manner that allows a user of the IWB appliance to view and edit the second data. The first data and the second data are also displayed on one or more other IWB appliances that are different than the IWB appliance, and at least a portion of the second data was received from the one or more other IWB appliances. For example, in the healthcare context, the first data may represent patient data and the second data may represent data pertaining to a healthcare facility, such as a room location of a patient, on-duty clinicians, etc., that is manually entered and updated over time by clinicians, administrative personnel, etc. Changes to the second data are automatically propagated to other IWB appliances.

Embodiments include a timeline view that allows users to view prior versions of the first and second data that correspond to particular dates and/or times. Embodiments also include support for user authentication, views, copy and paste of edited information, and mobile devices. The approach solves the problem of how to share and collaborate, via IWB appliances, on information that contains information subject to regulatory or legal requirements, such as health record information.

### II. System Architecture

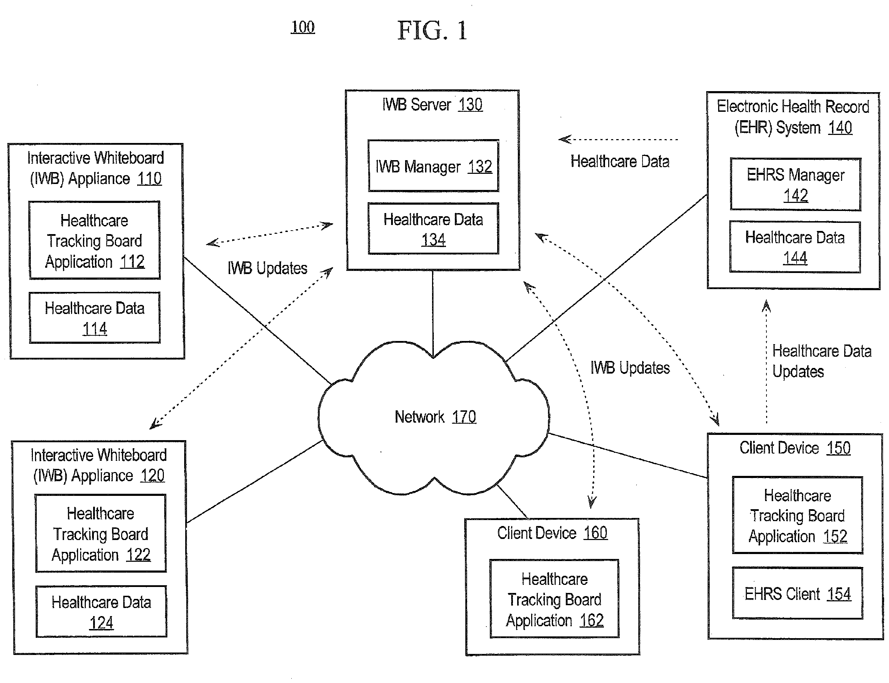

FIG. 1 is a block diagram that depicts an arrangement 100 for displaying information on IWB appliances. The particular elements depicted in arrangement are not all required, and embodiments are applicable to arrangement 100 having fewer elements or additional elements that may vary depending upon a particular implementation. Although embodiments are depicted in the figures and described herein in the healthcare context, this is done for explanation purposes and embodiments are not limited to the healthcare context.

### A. Overview

In the example depicted in FIG. 1, arrangement 100 includes IWB appliances 110, 120, an IWB server 130, an EHR system 140, and client devices 150, 160 communicatively coupled by a network 170. Arrangement 100 may include fewer or additional elements that may vary depending upon a particular implementation. Network 170 may be implemented by any number and types of networks that may vary depending upon a particular implementation. The elements depicted in FIG. 1 may also have direct communications links with each other and with other elements not depicted in FIG. 1.

### B. IWB Appliances

IWB appliances 110, 120 are electronic whiteboards that support interactive electronic whiteboard sessions with other IWB appliances. IWB appliances 110, 120 include various computer hardware and computer software elements that may vary depending upon a particular implementation, and embodiments are not limited to any particular computer hardware and computer software elements. Example elements include, without limitation, a display, which may be a touchscreen display, a communications interface, one or more processors, one or more memories, including volatile and non-volatile memory, an operating system (OS), and one or more applications including an IWB application that allows users to view and collaborate on information displayed on IWB appliances 110, 120.

According to one embodiment, IWB appliances 110, 120 include healthcare tracking board applications 112, 122, respectively, that allow users to view and collaborate on healthcare information. Healthcare tracking board applications 112, 122 may be implemented by one or more processes, plug-ins, library routines, etc., that may be stand-alone applications or functionality integrated into one or more other processes. Healthcare tracking board applications 112, 122 display healthcare-related information to users and receive user input via the displays of IWB appliances 110, 120. Healthcare tracking board applications 112, 122 may retrieve healthcare-related information from, and provide healthcare-related information to, IWB server 130 via requests that conform to an application program interface provide by IWB server 130. For example, IWB appliances 110, 120 may be deployed at different locations within a heath care facility and upon startup, healthcare tracking board applications 112, 122 request healthcare data from IWB server 130. Users of healthcare tracking board applications 112, 122 provide healthcare-related information, for example by writing information on the displays of IWB appliances 110, 120, and the information is propagated to the other IWB appliances 110, 120 and client devices 150, 160 via IWB server 130. For example, in response to detecting a change to healthcare-related information displayed on the display of IWB appliances 110, 120, healthcare tracking board applications 112, 122 may generate and transmit to IWB server 130 one or more update messages specifying the change made to healthcare-related information. Communications between IWB appliances 110, 120 and IWB server 130 may be in any format and in accordance with any type of communications protocol that may vary depending upon a particular implementation, and embodiments are not limited to any particular data format or communications protocol. Although embodiments are depicted in the figures and described herein in the context of information being propagated to IWB appliances 110, 120 and client devices 150, 160 via IWB server 130, embodiments are not limited to these examples and information may be propagated directly between IWB appliances 110, 120 and client devices 150, 160.

As described in more detail hereinafter, according to one embodiment, the healthcare-related information displayed on IWB appliances 110, 120 and client devices 150, 160 includes first data and second data, where the first data displayed in a display-only manner that allows a user to view, but not edit, the first data. According to one embodiment, the first data includes healthcare-related data from EHR system 140, and the healthcare-related data is displayed in a manner that complies with one or more regulatory or legal requirements, such as the Health Insurance Portability and Accountability Act (HIPPA). The second data is displayed concurrently with the first data, but in an editable manner that allows a user to view and edit the second data. For example, the first data may specify the names and locations of patients in a healthcare facility, and the second data may be additional information pertaining to the patients manually entered by clinicians of the health care facility, such as the name of an assigned clinician.

As described in more detail hereinafter, IWB appliances 110, 120 may maintain local healthcare data 114, 124, respectively, to allow IWB appliances 110, 120 to operate in stand-alone mode, for example, if communications with IWB server 130 and/or other IWB appliances are not available. Healthcare data 114, 124 may include one or more portions, or all, of healthcare data 134 maintained by IWB server 130.

### C. IWB Server

IWB server 130 manages data displayed by IWB appliances 110, 120 and client devices 150, 160, and more specifically, data displayed by and updated by healthcare tracking board applications 112, 122, 152, 162. IWB server 130 may be implemented by computer hardware, computer software, or any combination of computer hardware and computer software. For example, IWB server 130 may be implemented by one or more processes executing on one or more computing devices, such as network elements. IWB server 130 is depicted in FIG. 1 as a stand-alone element for purposes of explanation, but embodiments are not limited to this example and IWB server 130 may be implemented in other locations, for example, on IWB appliances 110, 120 or client devices 150, 160. In the example depicted in FIG. 1, IWB server 130 includes an IWB manager 132 and healthcare data 134. IWB sever 130 may provide an application program interface accessible by IWB appliances 110, 120 and client devices 150, 160 for storing and retrieving healthcare-related information to and from IWB server 130.

IWB manager 132 manages healthcare data on behalf of IWB appliances 110, 120 and client devices 150, 160. IWB manager 132 may receive healthcare data from EHR system 140 by request. For example, EHR system 140 may provide an application program interface that supports requests for healthcare data. Upon startup and/or at other times, IWB manager 132 issues a request to EHR system 140 for healthcare data for a particular healthcare facility, for one or more patients, etc. EHR system 140 processes the request and provides a response back to IWB manager 132 that includes the requested healthcare data.

IWB manager 132 may also receive healthcare data from EHR system 140 via messages issued by EHR system 140. For example, EHR system 140 may issue Health Level 7 (HL7) messages, such as admission, discharge and transfer (ADT) messages, in response to changes made to healthcare data 144. Example changes include, without limitation, patient check-in, patient check-out, change in assigned room or location, and any other changes to patient information. IWB manager 132 updates healthcare data 134 to include the healthcare data received from EHR system 140. Thus, IWB server 130 may obtain initial healthcare data from EHR system 140 that is subsequently updated by messages issued by EHR system 140.

IWB manager 132 processes requests for healthcare data received from healthcare tracking board applications 112, 122, 152, 162 by providing healthcare data back to healthcare tracking board applications 112, 122, 152, 162. For example, IWB manager 132 may receive requests for healthcare data from healthcare tracking board applications 112, 122, 152, 162 after startup and/or at other times. IWB manager 132 processes those requests by providing current healthcare data, from healthcare data 134, back to healthcare tracking board applications 112, 122, 152, 162.

IWB manager 132 also processes update requests received from healthcare tracking board applications 112, 122, 152, 162. The update requests may specify changes to healthcare data made by users of IWB appliances 110, 120 and client devices 150, 160. For example, IWB appliances 110, 120 may generate update requests in response to detected user input, such as a drawing or annotation on the display of IWB appliances 110, 120. IWB manager 132 processes the update requests by updating healthcare data 134 and propagating the specified changes to the other IWB appliances 110, 120 and client devices 150, 160. Thus, healthcare data 134 reflects all the changes made to healthcare data on IWB appliances 110, 120 and client devices 150, 160, subject to any latencies or outages in communications between IWB appliances 110, 120, client devices 150, 160 and IWB server 130.

According to one embodiment, IWB server 130 supports versioning of healthcare data. Versioning of healthcare data may be implemented using a wide variety of techniques and embodiments are not limited to any particular technique. According to one embodiment, an initial version of healthcare data 134 is established when healthcare data 134 is initially acquired from EHR system 140. Subsequent versions of healthcare data 134 are created when updates are received from EHR system 140 and/or from IWB appliances 110, 120 or client devices 150, 160. According to an embodiment, versions of healthcare data 134 are timestamped to support the timeline view feature described in more detail hereinafter. Version information may be stored as part of, or separate from, healthcare data 134.

### D. EHR System

EHR system 140 is a system for managing healthcare data. EHR system 140 may be implemented by computer hardware, computer software, or any combination of computer hardware and computer software. For example, EHR system 140 may be implemented by one or more processes executing on one or more computing devices, such as network elements. EHR system 140 is depicted in FIG. 1 as a stand-alone element for purposes of explanation, but embodiments are not limited to this example and EHR system 140 may be implemented in other locations, for example, on IWB server 130, IWB appliances 110, 120, and/or client devices 150, 160.

EHR system 140 may correspond to a particular healthcare entity, such as a hospital, healthcare network etc. Alternatively, EHR system 140 may serve multiple healthcare organizations. Although embodiments are depicted in the figures and described herein in the context of a single EHR system 140, this is done for explanation purposes only, and embodiments are applicable to any number of EHR systems.

According to one embodiment, EHR system 140 includes an EHRS manager 142 that manages healthcare data 144. This includes processing requests to update healthcare data 144 received from EHRS client 154 on client device 150. This also includes issuing update messages to IWB server 130, and possible one or more other devices and/or systems, in response to changes to healthcare data 144. As previously described herein, these messages may be HL7 messages, but other formats and/or protocols may be used. Healthcare data 144 may represent, for example, a master patient index.

### E. Client Devices

Client devices 150, 160 may be any type of client devices and examples of client devices 150, 160 include, without limitation, a workstation, a personal computer, a notebook or laptop computer, a tablet computing device, a personal digital assistant, and a smart phone. In the example depicted in FIG. 1, client devices 150, 160 include healthcare tracking board applications 152, 162, respectively, that allows users to view and participate in collaboration sessions with IWB appliances 110, 120 in the same manner as users of IWB appliances 110, 120. This may include, for example, viewing information displayed on IWB appliances 110, 120. In addition, changes made to the information displayed on IWB appliances 110, 120 are propagated to healthcare tracking board applications 152, 162 in the same manner as changes are propagated between IWB appliances 110, 120. Furthermore, changes to information made by users of client devices 150, 160 may be propagated to IWB appliances 110, 120. According to one embodiment, to provide enhanced security, healthcare tracking board application 152 on client 150 may be configured to limit the ability for a user to edit, including completely preventing the user from editing, the second data displayed by healthcare tracking board application 152. For example, both the first and second data may be displayed by healthcare tracking board application 152 in a display-only manner and controls for editing the second data may be disabled or not made available.

Client device 150 includes an EHRS client 154 that provides a graphical user interface to allow a user of client device 150 to update healthcare data 144 on EHR system 140. The graphical user interface may, for example, allow a user to search for a particular patient and then update data for that patient, such as patient check in information, patient check out information, change in assigned room or location, and any other patient information. Similar to healthcare tracking board applications 112, 122, healthcare tracking board applications 152, 162 may be implemented by one or more processes, plug-ins, library routines, etc., that may be stand-alone applications or functionality integrated into one or more other processes.

### III. Displaying Information on IWB Appliances

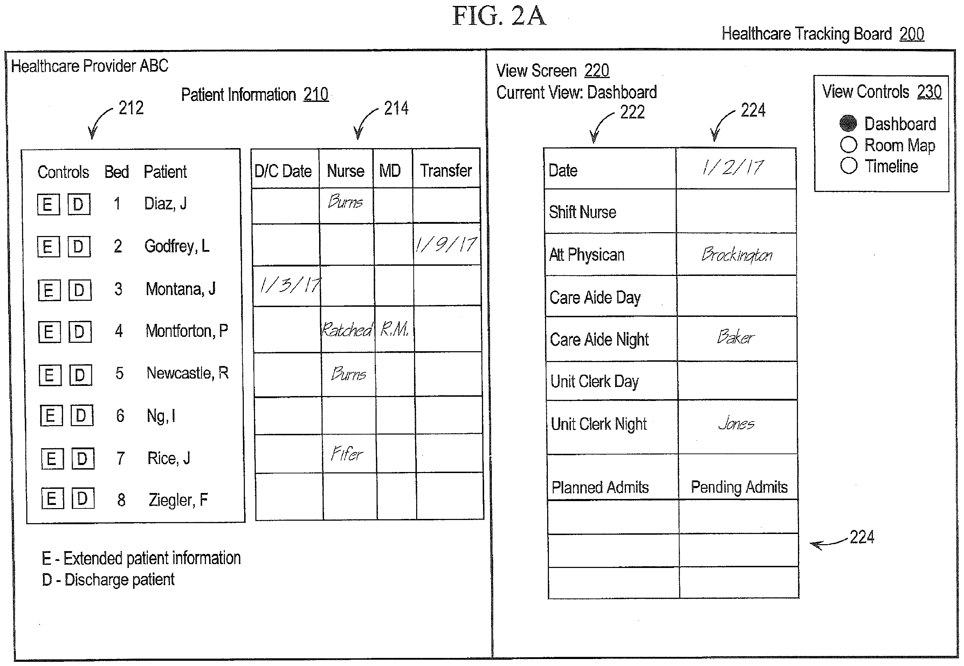

FIG. 2A depicts an example graphical user interface screen, in the form of a healthcare tracking board 200, generated by healthcare tracking board applications 112, 122, 152, 162 for displaying healthcare information. In the example depicted in FIG. 2A, healthcare tracking board 200 is provided for a healthcare provider named "Healthcare Provider ABC," which could be any type of healthcare entity, for example, a hospital, clinic, etc. Access to healthcare tracking board 200 may be conditioned upon a user having a required authorization. For example, an employee may be required to provide user credentials, for example, manually, or via an identification badge, RFID tag, etc., and the provided user credential verified, prior to a user being granted access to healthcare tracking board 200.

Healthcare provider board 200 may be implemented using a wide variety of techniques that may vary depending upon a particular implementation, and embodiments are not limited to any particular technique. For example, healthcare provider board 200 may be implemented using global and local windows in which information in global windows is shared with and propagated to other IWB appliances and client devices, and in which information in local windows is displayed on only a particular IWB appliance or client device.

In the example depicted in FIG. 2A, healthcare provider board 200 includes patient information 210 and a view screen 220. Patient information 210 includes first data 212 that is provided by EHR system 140 and is displayed in a display-only, i.e., read-only, manner, meaning that users can view, but not edit, first data 212. In the example depicted in FIG. 2A, first data 212 identifies current patients and their bed locations. First data 212 also includes controls that allow a user to perform one or more actions with respect to a particular patient. An "E" control allows a user to view extended patient information, such as a full patient record, and a "D" control allows a user to discharge a patient. Access to these controls may be conditioned upon a user having a required authorization. For example, access to the controls may be limited to physicians and a head nurse. In addition, selection of the "E" control to view extended patient information may cause the extended information to be displayed at only the IWB appliance 110, 120, or client device 150, 160 where the selection was made, and not on other IWB appliances or client devices. This prevents unauthorized access to the extended patient information. Selection of the "D" control to discharge a patient may cause an immediate update on the displays of all of the IWB appliances 110, 120 and client devices 150, 160. Implementing the discharge of the patient in EHR system 140 is not immediately made in response to the selection of the "D" control, and is instead implemented by a user of client device 150, as described in more detail hereinafter. Therefore, when the "D" control is selected for a patient, a visual indication may be provided on the displays of IWB appliances 110, 120 and client devices 150, 160 to indicate that a patient has been discharged, and to provide a notification of the user of client device 150 that EHR system 140 should be updated.

According to one embodiment, first data 212 is displayed in a manner that complies with a regulatory or legal requirement, such as HIPPA. This may include, for example, displaying a limited amount of patient information, e.g., abbreviated names, etc., and/or in a particular manner, e.g., by obscuring portions of patient information. For example, patient names may be displayed in abbreviated form, and without personal information such as social security numbers, patient-specific health information, etc. According to an embodiment, controls are provided for selecting a manner in which first data 212 is displayed. For example, controls may be provided for displaying first data 212 in a public mode, i.e., in accordance with a regulatory or legal requirement, such as HIPPA, or displaying first data 212 in a private mode, that does not display the first data 212 in accordance with such as a requirement.

Patient information 210 also includes second data 214 that is displayed concurrently with the first data 212 and in an editable manner that allows users of the healthcare tracking board applications 112, 122, 152, 162 to enter and edit the second data 214. Users may enter and edit the second data 214 using any of a wide variety of methods that may vary depending upon a particular implementation, and embodiments are not limited to any particular method for updating second data 214. Example methods include, without limitation, handwriting using a stylus or pointing device, such as a mouse, trackball or keyboard, or by hand via a touch display of IWB appliances 110, 120, or client devices 150, 160. Entry and editing of second data 214 may be subject to a user having sufficient permission to do so. For example, healthcare tracking board applications 112, 122, 152, 162 may require a user to be properly authorized to enter and edit second data 214.

Healthcare tracking board applications 112, 122, 152, 162 may use various techniques for improving accuracy and readability. For example, healthcare tracking board applications 112, 122, 152, 162 may incorporate text recognition to recognize hand written text, which may include real-time spell checking functionality that provides automatic spelling updates and/or suggested spelling and/or words and allows a user to confirm a suggestion. This helps to improve the accuracy and readability of information manually provided by users. In addition, functionality from other applications may be integrated into healthcare tracking board 200. For example, calendars may be used to specify discharge (D/C) dates, and nurses and/or physicians (MDs) may be selected from lists that are provided by other applications and/or systems. Users in multiple locations may change the second data 214 and the changes are propagated to the other IWB appliances 110, 120, and client devices 150, 160, either automatically, or in response to a user request.

According to one embodiment, controls are provided on healthcare tracking board 200 to allow a user to specify whether changes are to be automatically propagated to other IWB appliances 110, 120 and client devices 150, 160. For example, the default value for such a control may specify that changes are to be automatically propagated to other IWB appliances 110, 120 and client devices 150, 160, e.g., via a global window, while another value for the control does not propagate the changes to other IWB appliances 110, 120 and client devices 150, 160, but instead displays the changes only locally, e.g., via a local window. In the example depicted in FIG. 2A, the second data 214 includes, for each patient, a discharge date, an assigned nurse, an assigned physician, and a transfer date. Embodiments are not limited to the particular example of second data 214 depicted in FIG. 2A and any type or amount of second data 214 may be used.

According to one embodiment, healthcare tracking board applications 112, 122, 152, 162 include "copy & paste" functionality that allows a user to select, copy and paste one or more portions, including all, of the second data 214. For example, a user may select nurse "Burns" assigned to patient "Diaz, J" and copy and paste nurse "Bums" to one or more other locations, for example, to other patients in second data 214. One or more portions of the second data 214 may be copied and pasted to other locations, including second data 224 of view screen 220 described in more detail hereinafter. The other locations may also include external locations, such as documents, e.g., word processing documents, spreadsheets, etc., files, network locations, etc., to allow edited second data 214 to be easily saved. This allows a user, for example, to easily save the entire second data 214 into a document for safekeeping.

View screen 220 displays a current view to a user. In the example depicted in FIG. 2A, the current view is a dashboard view that displays information pertaining to the healthcare facility. While patient information 210 generally displays patient-specific information, the dashboard view generally provides non-patient-specific information, although this is not required and the particular contents of view screen 220 may vary depending upon a particular implementation. In the example depicted in FIG. 2A, the dashboard view displays information about the healthcare facility and includes first data 222 and second data 224. Like the first data 212 of patient information 210, first data 222 is displayed in a display-only, i.e., read-only, manner, meaning that users cannot edit the first data 222. Unlike first data 212, which is patient specific and provided by EHR system 140, first data 222 is not patient specific and does not need to be supplied by EHR system 140. Instead, being specific only to a particular healthcare facility, first data 222 may be maintained by IWB server 130, or another location that manages data for the particular healthcare facility. In the example depicted in FIG. 2A, first data 222 identifies a current date, a shift nurse, an attending physician, a care aide day, a care aide night, a unit clerk day, a unit clerk night, planned admits and pending admits. Embodiments are not limited to the particular example depicted in FIG. 2A and the contents of first data 222 may vary depending upon a particular implementation. Other aspects of first data 212 may be applied to first data 222, depending upon a particular implementation.

Second data 224 is displayed concurrently with the first data 212 and in an editable manner that allows users of the healthcare tracking board applications 112, 122, 152, 162 to enter and edit the second data 224 in the same manner as second data 214. In the example depicted in FIG. 2A, second data 224 includes user-specified values for the current date, the shift nurse, the attending physician, the care aide day, the care aide night, the unit clerk day, the unit clerk night, the planned admits and the pending admits. As depicted in FIG. 2A, values are not required for all of the items in second data 214 and second data 224. First data 212, 222 and second data 214, 224 may be displayed in a manner to visually indicate that first data 212, 222 are display only and second data 214, 224 are editable, for example, using colors, shading, special effects, etc.

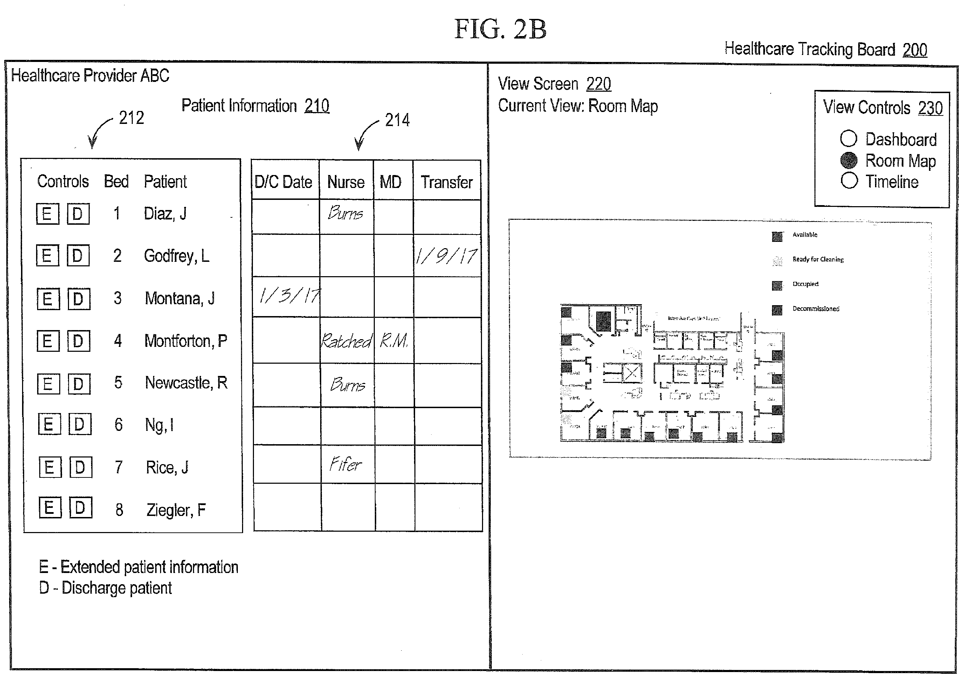

View controls 230 allow a user to change views. In the example depicted in FIG. 2A, controls are provided for switching between views, but embodiments are not limited to this example, and any number and types of views may be provided and vary depending upon a particular implementation. View controls 230 allow the user to change from the current dashboard view to a "Room Map" view by selecting the corresponding radial button. FIG. 2B depicts a "Room Map" view in response to a user selecting the "Room Map" control from view controls 230. The "Room Map" view depicts the physical locations, e.g., room assignments, of patients within a healthcare facility and may be useful to quickly identify available rooms, etc. Rooms may be visually depicted in a manner to identify whether they are occupied or available, for example, using colors, shading, special effects, etc. Embodiments are not limited to the example depicted in FIG. 2B and the particular content, arrangement and "look and feel" of a room map may vary depending upon a particular implementation. Embodiments are also not limited to the example views depicted in the figures and described herein. Examples of other views that may be implemented included, without limitation, public and private views, and views that correspond to a particular logical group, such as a department of a business organization, a project, team, etc. For example, view controls 230 may include controls for a plurality of departments within a healthcare service provider, such as a hospital. Users may then select a view for a particular department and view the first data and second data for the particular view. Views may also be customized for particular users and access to customized views may be conditioned upon a successful authentication of a particular user. For example, a clinician may select a view that is particular to that clinician and includes data that is only relevant to the clinician.

### IV. Timeline Views

According to one embodiment, healthcare tracking board applications 112, 122, 152, 162 are configured to store, and make available to users, healthcare tracking board 200 at different points in time. Healthcare tracking board 200 data may be saved in different ways that may vary depending upon a particular implementation, and embodiments are not limited to any particular approach for saving healthcare tracking board 200 data. For example, healthcare tracking board 200 data may be automatically saved at specified intervals, such as per minute, per hour, per shift, etc., or in response to explicit user requests, e.g., via a control provided on healthcare tracking board 200. As another example, healthcare tracking board 200 data may be automatically saved in response to changes made to healthcare tracking board 200 data, so that all changes to healthcare tracking board 200 data are preserved. As yet another example, healthcare tracking board 200 data may be saved in response to a user request, alone or in combination with saving healthcare tracking board 200 data based upon time or in response to changes. The manner in which healthcare tracking board 200 data is saved may be configured individually at healthcare tracking board applications 112, 122, 152, 162. Healthcare tracking board 200 data may be stored in healthcare data 134 and/or in healthcare data 114, 124 and may be timestamped, i.e., saved with data that specifies a date and/or time for the saved healthcare tracking board 200 data.

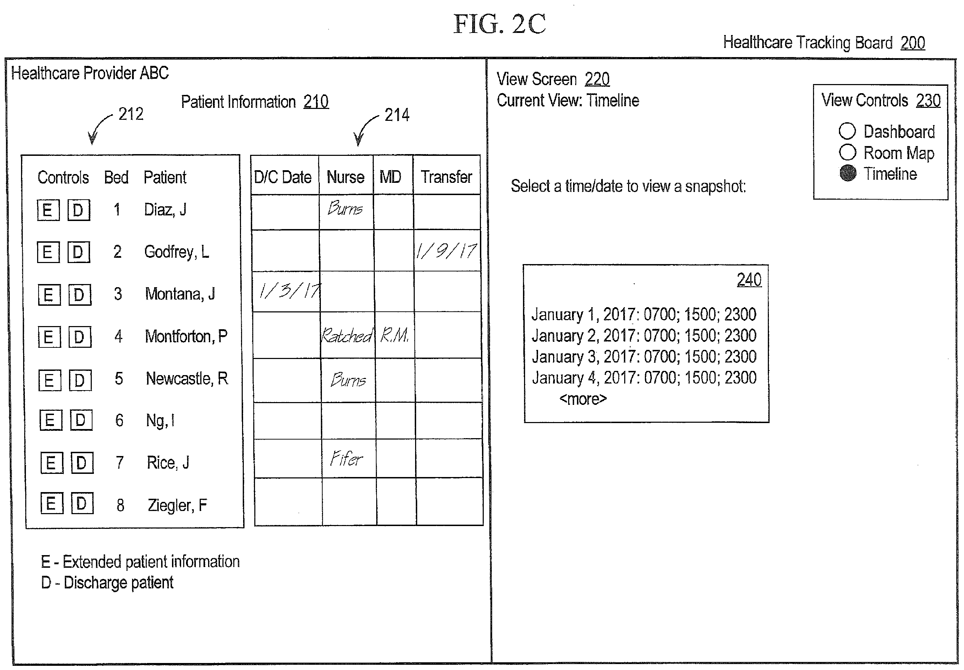

As depicted in FIG. 2A, view controls 230 allow a user to select a "Timeline View" option that provides user access to historical healthcare tracking board 200 data. FIG. 2C depicts healthcare tracking board 200 in response to a user selecting the "Timeline View" option from view controls 230. In FIG. 2C, view screen 220 depicts a date/time box 240 that displays available dates/times that may be selected by the user. The dates/times correspond to dates/times at which the healthcare tracking board 200 data was saved. In the example depicted in FIG. 2C, three times are available for each day, corresponding to the start of an eight-hour shift in a healthcare facility. Embodiments are not limited to the example depicted in FIG. 2C and any number of days and/or times may be used. According to one embodiment, a query box and/or calendar is provided to allow a user to specify a particular date/time that the user would like to view.

In response to a user selection or specification of a particular day/time, a snapshot of the healthcare tracking board 200 at the selected day/time is retrieved and displayed. For example, suppose that a user of healthcare tracking board application 112 selects the "January 1, 2017: 1500" date/time. In response to this selection, healthcare tracking board application 112 retrieves and displays the healthcare tracking board 200 as of January 1, 2017, at 1500 hours. Healthcare tracking board application 112 retrieves the healthcare tracking board 200 data from healthcare data 114 and/or from healthcare data 134. The healthcare tracking board 200 may be displayed in the same manner as depicted in FIG. 2A and displays all of the changes made via IWB appliance 110, 120 and client devices 150, 160 at the specified day and/or time. In addition, healthcare tracking board 200 may include data for any number of the views that were available at that time, e.g., the "Dashboard," "Room Map" and "Timeline" views. Thus, the timeline view allows a user to quickly and easily view historical snapshots of healthcare tracking board 200, which may be useful in a wide variety of contexts. For example, historical healthcare tracking board 200 data may be used to improve the management and operation of a healthcare facility, and/or to satisfy regulatory or legal requirements. In addition, the copy and paste functionality previously described herein may be used to copy information from a prior version of healthcare tracking board 200 and paste the information into the current healthcare tracking board 200.

### V. Updating Healthcare Data on EHR System

As previously described herein, the information displayed by healthcare tracking board applications 112, 122, 152, 162 includes first data that is displayed in a display-only manner and second data 214 that is displayed concurrently with the first data and in an editable manner. As previously described herein, healthcare tracking board application 152 may be configured to limit the ability of a user to edit, including completely preventing the user from editing, the second data displayed by healthcare tracking board application 152. According to one embodiment, updates to the second data are immediately propagated to IWB appliances 110, 120 and client devices 150, 160 to allow users of the healthcare tracking board applications 112, 122, 152, 162 to view the changes. Changes to the second data are not immediately propagated to EHR system 140 and instead are made by a user of client device 150.

As previously described herein, client device 150 includes an EHRS client 154 that provides access to healthcare data 144 via EHRS manager 142. EHRS client 154 provides a graphical user interface that allows a user of client device 150 to update healthcare data 144 on EHR system 140. The graphical user interface may, for example, allow a user to search for a particular patient and then update data for that patient, such as patient check in information, patient check out information, change in assigned room or location, and any other patient information. The user may be, for example, an administrative user that views changes made to second data displayed on the display of client device 150, via healthcare tracking board application 152, and then implements those changes in healthcare data 144 via EHRS client 154. EHRS manager 142, in conjunction with EHRS client 154, may require that a user be authenticated and have any necessary permissions prior to allowing the user to make changes to healthcare data 144. One of the benefits of this approach is that the user is able to see, via healthcare tracking board application 152, all of the changes made on IWB appliances 110, 120 and client device 150, and then update EHR system 140 with all of the changes. According to one embodiment, first data 212, 222 may be displayed in a manner that allows a user to specify changes to be made to first data 212, 222. For example, a user may be allowed to write over, or add instructions and/or notes to, first data 212, 222 to indicate a change that should be made to EHR system 140, to make the changes more conspicuous to the user of client device 150. As one example, a particular patient may be assigned to a particular room and a user of IWB appliance 110 crosses out the particular patient and writes in another patient. This lets users of IWB appliances 110, 120 and client devices 150, 160 immediately know that a different patient has been put into the particular room, and that the EHR system 140 should be updated with this change.

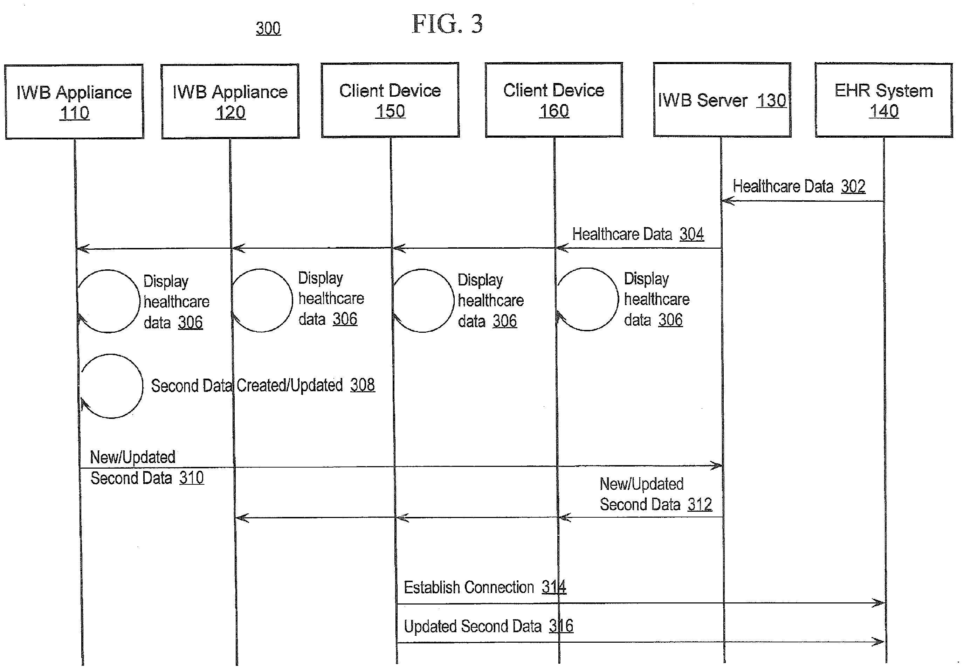

FIG. 3 depicts a message ladder diagram 300 that shows example message flows between the various elements of FIG. 1. In step 302, EHR system 140 provides healthcare data to IWB server 130. For example, EHR system 140 may issue messages, such as HL7 messages, that specify updates to healthcare data 144. The messages may be issued in response to changes made to healthcare data 144, such as patient check-in, patient check-out, a change in an assigned room or location, or any other changes to patient information. In step 304, IWB server 130 provides healthcare data to one or more of IWB appliances 110, 120, and client devices 150, 160. For example, upon startup, healthcare tracking board applications 112, 122, 152, 162 may request healthcare data from IWB server 130 to populate healthcare tracking board 200. In step 306, one or more of IWB appliance 110, 120, and client devices 150, 160 display the healthcare data received from IWB server 130. For example, one or more of healthcare tracking board applications 112, 122, 152, 162 may display the healthcare data as first data 212 on healthcare tracking board 200.

In step 308, second data is created and/or updated at one or more of IWB appliance 110, 120, and client devices 150, 160. For example, a user of IWB appliance 110 may enter and/or update second data 214, 224 via healthcare tracking board 200 displayed by healthcare tracking board application 112. This may be done, for example, via handwriting using a stylus or pointing device, such as a mouse, trackball or keyboard, or by hand via a touch display of IWB appliance 110. This may include entering new second data and/or updating existing second data. Healthcare tracking board application 112 may update local healthcare data 114 to include the new and/or updated healthcare data.

In step 310, the new or updated second data is provided to IWB server 130. For example, healthcare tracking board application 112 may provide the new and/or updated second data to IWB server 130. This may be done automatically, for example, when the new and/or updated second data was entered into healthcare tracking board 200. IWB server 130 updates healthcare data 134 to include the new and/or updated second data. According to one embodiment, IWB server 130 maintains versions of healthcare data 134 to support the timeline view feature previously described herein.

In step 312, the new and/or updated second data is propagated to one or more other devices. For example, IWB server 130 may provide the new and/or updated second data to one or more of IWB appliance 120, and client devices 150, 160. In response to receiving the new and/or updated second data, healthcare tracking board applications 122, 152, 162 display the new and/or updated second data 214, 224 on healthcare tracking board 200. Steps 308, 310 may be repeated for any amount of second data 214, 224 and/or changes made via healthcare tracking board 200. This allows changes in healthcare data to be automatically propagated to any number and types of devices so that users of the devices may immediately see the changes. For example, a user at IWB appliance 110 may change a nurse assigned to a particular patient and the change is immediately displayed at one or more other devices, such as IWB appliance 120, and client devices 150, 160.

In step 314, a user of client device 150 establishes a connection with EHR system 140. For example, an administrative user may use EHRS client 154 to connect to EHRS manager 142. In step 316, EHR system 140 is updated with the new and/or updated second data. For example, the user of client device 150 may use the EHRS client 154 to update healthcare data 144 with the new and/or updated second data.

### VI. Implementation Examples

Although the flow diagrams of the present application depict a particular set of steps in a particular order, other implementations may use fewer or more steps, in the same or different order, than those depicted in the figures.

According to one embodiment, the techniques described herein are implemented by one or more special-purpose computing devices. The special-purpose computing devices may be hard-wired to perform the techniques, or may include digital electronic devices such as one or more application-specific integrated circuits (ASICs) or field programmable gate arrays (FPGAs) that are persistently programmed to perform the techniques, or may include one or more general purpose hardware processors programmed to perform the techniques pursuant to program instructions in firmware, memory, other storage, or a combination. Such special-purpose computing devices may also combine custom hard-wired logic, ASICs, or FPGAs with custom programming to accomplish the techniques. The special-purpose computing devices may be desktop computer systems, portable computer systems, handheld devices, networking devices or any other device that incorporates hard-wired and/or program logic to implement the techniques.

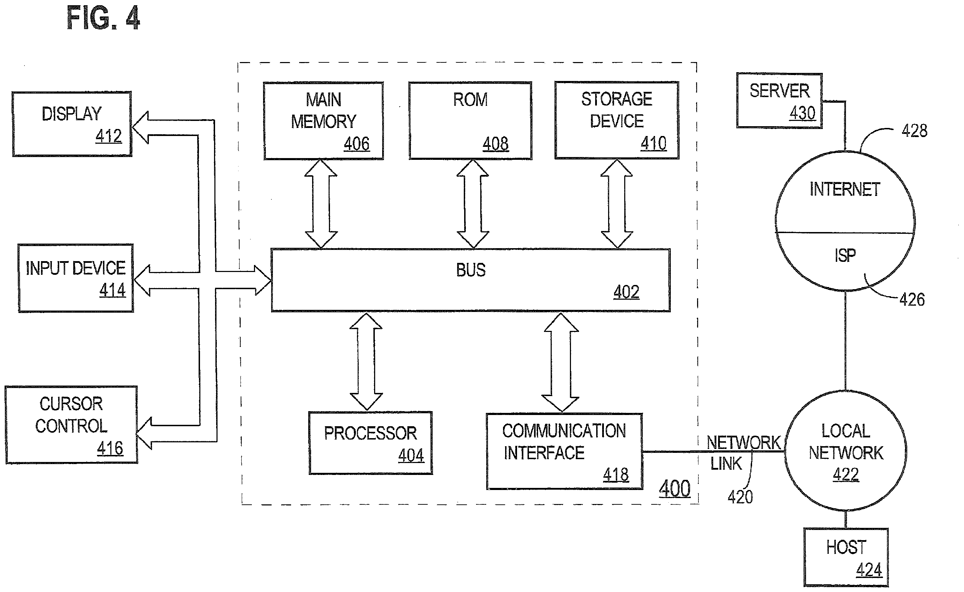

FIG. 4 is a block diagram that depicts an example computer system 400 upon which embodiments may be implemented. Computer system 400 includes a bus 402 or other communication mechanism for communicating information, and a processor 404 coupled with bus 402 for processing information. Computer system 400 also includes a main memory 406, such as a random access memory (RAM) or other dynamic storage device, coupled to bus 402 for storing information and instructions to be executed by processor 404. Main memory 406 also may be used for storing temporary variables or other intermediate information during execution of instructions to be executed by processor 404. Computer system 400 further includes a read only memory (ROM) 408 or other static storage device coupled to bus 402 for storing static information and instructions for processor 404. A storage device 410, such as a magnetic disk or optical disk, is provided and coupled to bus 402 for storing information and instructions.

Computer system 400 may be coupled via bus 402 to a display 412, such as a cathode ray tube (CRT), for displaying information to a computer user. Although bus 402 is illustrated as a single bus, bus 402 may comprise one or more buses. For example, bus 402 may include without limitation a control bus by which processor 404 controls other devices within computer system 400, an address bus by which processor 404 specifies memory locations of instructions for execution, or any other type of bus for transferring data or signals between components of computer system 400.

An input device 414, including alphanumeric and other keys, is coupled to bus 402 for communicating information and command selections to processor 404. Another type of user input device is cursor control 416, such as a mouse, a trackball, or cursor direction keys for communicating direction information and command selections to processor 404 and for controlling cursor movement on display 412. This input device typically has two degrees of freedom in two axes, a first axis (e.g., x) and a second axis (e.g., y), that allows the device to specify positions in a plane.

Computer system 400 may implement the techniques described herein using customized hard-wired logic, one or more ASICs or FPGAs, firmware and/or program logic or computer software which, in combination with the computer system, causes or programs computer system 400 to be a special-purpose machine. According to one embodiment, those techniques are performed by computer system 400 in response to processor 404 processing instructions stored in main memory 406. Such instructions may be read into main memory 406 from another non-transitory computer-readable medium, such as storage device 410. Processing of the instructions contained in main memory 406 by processor 404 causes performance of the functionality described herein. In alternative embodiments, hard-wired circuitry may be used in place of or in combination with software instructions to implement the embodiments. Thus, embodiments are not limited to any specific combination of hardware circuitry and software.

The term "non-transitory computer-readable medium" as used herein refers to any non-transitory medium that participates in providing data that causes a computer to operate in a specific manner. In an embodiment implemented using computer system 400, various computer-readable media are involved, for example, in providing instructions to processor 404 for execution. Such media may take many forms, including but not limited to, non-volatile and volatile non-transitory media. Non-volatile non-transitory media includes, for example, optical or magnetic disks, such as storage device 410. Volatile non-transitory media includes dynamic memory, such as main memory 406. Common forms of non-transitory computer-readable media include, without limitation, a floppy disk, a flexible disk, hard disk, magnetic tape, or any other magnetic medium, a CD-ROM, any other optical medium, a RAM, a PROM, and EPROM, a FLASH-EPROM, any other memory chip, memory cartridge or memory stick, or any other medium from which a computer can read.

Various forms of non-transitory computer-readable media may be involved in storing instructions for processing by processor 404. For example, the instructions may initially be stored on a storage medium of a remote computer and transmitted to computer system 400 via one or more communications links. Bus 402 carries the data to main memory 406, from which processor 404 retrieves and processes the instructions. The instructions received by main memory 406 may optionally be stored on storage device 410 either before or after processing by processor 404.

Computer system 400 also includes a communication interface 418 coupled to bus 402. Communication interface 418 provides a communications coupling to a network link 420 that is connected to a local network 422. For example, communication interface 418 may be a modem to provide a data communication connection to a telephone line. As another example, communication interface 418 may be a local area network (LAN) card to provide a data communication connection to a compatible LAN. Wireless links may also be implemented. In any such implementation, communication interface 418 sends and receives electrical, electromagnetic or optical signals that carry digital data streams representing various types of information.

Network link 420 typically provides data communication through one or more networks to other data devices. For example, network link 420 may provide a connection through local network 422 to a host computer 424 or to data equipment operated by an Internet Service Provider (ISP) 426. ISP 426 in turn provides data communication services through the world wide packet data communication network now commonly referred to as the "Internet" 428. Local network 422 and Internet 428 both use electrical, electromagnetic or optical signals that carry digital data streams.

Computer system 400 can send messages and receive data, including program code, through the network(s), network link 420 and communication interface 418. In the Internet example, a server 430 might transmit a requested code for an application program through Internet 428, ISP 426, local network 422 and communication interface 418. The received code may be processed by processor 404 as it is received, and/or stored in storage device 410, or other non-volatile storage for later execution.

In the foregoing specification, embodiments have been described with reference to numerous specific details that may vary from implementation to implementation. Thus, the sole and exclusive indicator of what is, and is intended by the applicants to be, the invention is the set of claims that issue from this application, in the specific form in which such claims issue, including any subsequent correction. Hence, no limitation, element, property, feature, advantage or attribute that is not expressly recited in a claim should limit the scope of such claim in any way. The specification and drawings are, accordingly, to be regarded in an illustrative rather than a restrictive sense.

The present application is based on United States priority application No. 15/476,186 filed on March 31, 2017, the entire contents of which are hereby incorporated herein by reference.

## Claims

1. An interactive whiteboard (IWB) appliance comprising:
a display;
one or more processors; and
a tracking board application configured to:
cause first data to be displayed on the display of the IWB appliance in a display-only manner that allows a user of the IWB appliance to view, but not edit, the first data,
cause second data to be displayed on the display of the IWB appliance, concurrently with the first data, in an editable manner that allows the user of the IWB appliance to view and edit the second data,
wherein the first data and the second data are displayed on one or more other IWB appliances that are different than the IWB appliance, and at least a portion of the second data was received by the IWB appliance from the one or more other IWB appliances.

2. The IWB appliance of Claim 1, wherein the tracking board application is further configured to:
cause to be displayed, on the display of the IWB appliance, one or more timeline controls that allow a user to select a prior time from a plurality of prior times,
in response to a user selection of a particular prior time from the plurality of prior times:
retrieving a prior version of the first data and a prior version of the second data that correspond to the particular prior time,
causing the prior version of the first data to be displayed on the display of the IWB appliance in the display-only manner that allows the user of the IWB appliance to view, but not edit, the prior version of the first data, and
causing the prior version of the second data to be displayed on the display of the IWB appliance, concurrent with the display of the prior version of the first data on the display of the IWB appliance, in an editable manner that allows a user of the IWB appliance to view and edit the prior version of the second data.

3. The IWB appliance of Claim 1, wherein:
the first data includes a plurality of selectable data items, and
the tracking board application is further configured to, in response to a user selection of a particular selectable data item from the plurality of selectable data items included in the first data:
determine whether the user is authorized to access the particular selectable data item, and
in response to determining that the user is authorized to access the particular selectable data item, retrieving and displaying on the display of the IWB appliance, additional information that corresponds to the particular selectable data item.

4. The IWB appliance of Claim 1, wherein:
the tracking board application is a healthcare tracking board application, and
the first data is healthcare data retrieved from an electronic health record system.

5. The IWB appliance of Claim 4, wherein the first data is displayed in a manner that complies with a regulatory or legal requirement that specifies one or more limitations on displaying personal information.

6. The IWB appliance of Claim 1, wherein the tracking board application is further configured to:
detect a change made to the second data, and
in response to detecting the change made to the second data, perform one or more of: update healthcare data stored on the IWB appliance to include the change made to the second data, or propagate the detected change made to the second data to the one or more other IWB appliances by generating and transmitting an update request to an IWB server.

7. The IWB appliance of Claim 1, wherein the tracking board application is further configured to:
display one or more view controls that provide user access to a plurality of views that include at least a timeline view and a room map view, wherein the timeline view provides access to prior versions of the first data and the second data at a plurality of prior times, and the room map view visually depicts patient room assignments.

8. A computer-implemented method comprising:
a tracking board application executing on an interactive whiteboard (IWB) appliance:
causing first data to be displayed on a display of the IWB appliance in a display-only manner that allows a user of the IWB appliance to view, but not edit, the first data,
causing second data to be displayed on the display of the IWB appliance, concurrently with the first data, in an editable manner that allows the user of the IWB appliance to view and edit the second data,
wherein the first data and the second data are displayed on one or more other IWB appliances that are different than the IWB appliance, and at least a portion of the second data was received by the IWB appliance from the one or more other IWB appliances.

9. The computer-implemented method of Claim 8, further comprising the tracking board application:
causing to be displayed, on the display of the IWB appliance, one or more timeline controls that allow a user to select a prior time from a plurality of prior times,
in response to a user selection of a particular prior time from the plurality of prior times:
retrieving a prior version of the first data and a prior version of the second data that correspond to the particular prior time,
causing the prior version of the first data to be displayed on the display of the IWB appliance in the display-only manner that allows the user of the IWB appliance to view, but not edit, the prior version of the first data, and
causing the prior version of the second data to be displayed on the display of the IWB appliance, concurrent with the display of the prior version of the first data on the display of the IWB appliance, in an editable manner that allows a user of the IWB appliance to view and edit the prior version of the second data.

10. The computer-implemented method of Claim 8, wherein:
the first data includes a plurality of selectable data items, and
the computer-implemented method further comprises the tracking board application, in response to a user selection of a particular selectable data item from the plurality of selectable data items included in the first data:
determining whether the user is authorized to access the particular selectable data item, and
in response to determining that the user is authorized to access the particular selectable data item, retrieving and displaying on the display of the IWB appliance, additional information that corresponds to the particular selectable data item.

11. The computer-implemented method of Claim 8, wherein:
the tracking board application is a healthcare tracking board application, and
the first data is healthcare data retrieved from an electronic health record system.

12. The computer-implemented method of Claim 11, wherein the first data is displayed in a manner that complies with a regulatory or legal requirement that specifies one or more limitations on displaying personal information.

13. The computer-implemented method of Claim 8, further comprising the tracking board application:
detecting a change made to the second data, and
in response to detecting the change made to the second data, performing one or more of:
updating healthcare data stored on the IWB appliance to include the change made to the second data, or propagating the detected change made to the second data to the one or more other IWB appliances by generating and transmitting an update request to an IWB server.

14. The computer-implemented method of Claim 8, further comprising the tracking board application:
displaying one or more view controls that provide user access to a plurality of views that include at least a timeline view and a room map view, wherein the timeline view provides access to prior versions of the first data and the second data at a plurality of prior times, and the room map view visually depicts patient room assignments.

15. One or more non-transitory computer-readable media storing instructions which, when executed by one or more processors, perform the computer-implemented method of any one of claims 8-14.
